# EUROPEAN PATENT APPLICATION

(11) **EP 4 140 415 A1**
(43) Date of publication of application: **01.03.2023**
(21) Application number: 21199080.9
(22) Date of filing: 27.09.2021
(51) Int. Cl.: A61B 8/08

(54) **METHOD FOR USE IN ANALYSING ULTRASOUND IMAGE DATA OF A SUBJECT**

(30) Priority: 27.08.2021 WO PCT/CN2021/114874
(71) Applicant: Koninklijke Philips N.V., 5656 AG Eindhoven (NL)
(72) Inventor: CHEN, Zhouye, Eindhoven (NL); LIN, Qi Zhong, Eindhoven (NL); HONG, Xiaowei, Eindhoven (NL); TONG, Ling, Eindhoven (NL); DENG, Jun Ping, Eindhoven (NL); XU, Jing Ping, Eindhoven (NL); LI, Happy L, Eindhoven (NL); ZHAO, Ning Ning, Eindhoven (NL)
(74) Representative: Philips Intellectual Property & Standards

(57) **Abstract**

According to an aspect, there is provided an apparatus for use in analysing ultrasound image data of a subject, the apparatus comprising a memory comprising instruction data representing a set of instructions; and a processor configured to communicate with the memory and to execute the set of instructions, wherein the set of instructions, when executed by the processor, cause the processor to: obtain contrast-enhanced ultrasound image data comprising a plurality of frames; estimate at least one time point corresponding to at least one event; select a sample of frames from the plurality of frames based on the at least one estimated time point; and classify each of the sample of frames.

## Description

### FIELD OF INVENTION

The invention relates to methos and apparatuses for use in analysing ultrasound image data of a subject, and more particularly, for analysing contrast enhanced ultrasound images of subject.

### BACKGROUND OF THE INVENTION

Ultrasound, especially contrast enhanced ultrasound (CEUS), has become increasingly widely used for medical imaging diagnosis as it is relative cheap, fast and non-invasive, when compared with computerised tomography (CT) and magnetic resonance imaging (MRI) which require complex instruments and have high costs associated with them. CEUS offers several advantages over other forms of medical imaging, including high diagnostic accuracy and lower costs, as well as fewer side effects. One particular use of CEUS is in the in diagnosis of a liver lesion. Contrast-enhanced ultrasound (CEUS) is an important imaging technique used for assessing liver disease including focal liver lesions (FLLs), and provides the advantages of enabling real-time imaging and quantification of FLL perfusion.

During the process for diagnosis of a liver lesion using CEUS images, a doctor injects a contrast agent into the blood vessels of a patient and images a region of the liver (including the liver lesion) using ultrasound. As the contrast agent flows along with blood in the body, the CEUS images will form three main phases - namely an arterial phase, a portal venous phase, and a delayed phase. The duration and imaging features of each phase are different. A clinician analysing the CEUS images taken may make a diagnosis based on their observation of the characteristics of the lesion in these phases.

### SUMMARY OF THE INVENTION

The process currently used for a clinician to analyse CEUS images to make a diagnosis involves playing a CEUS loop of the CEUS images back and forth to interpret and extract features or parameters that they require, where the features or parameters may be found with reference to a template. The template typically comprises features or parameters which are used for diagnosis of a disease. Different diseases have different templates. As example, to diagnose a liver lesion, a liver template which comprises features usable for liver lesion diagnosis is selected from a template pool of various templates such as a liver template, breast template, thyroid template and so on. Some hospitals use customized templates which are composed of a selection of features which could be used for liver lesion diagnosis. In an example of such a template, all features for liver lesion diagnosis are listed as checklist, and a customized template is created using the checklist. The template may then be filled in to form the report. The most time-consuming part of this process, especially for less experienced clinicians, is the extraction of the feature or parameter from the CEUS images. The process of feature extraction may include finding an optimal reference frame, where the frame presents sufficiently well-distinguished lesion from its surrounding tissue, by visual observation. The process may additionally include manually adding markers which may be used to denote the long and short axis of lesion on the optimal reference frame.

In addition, the process may further require a clinician monitoring the relative changes of brightness intensity of both a lesion and its surrounding background tissue over time in order to extract particular features. However, there is often low, or no obvious, contrast between the lesion and its surrounding background tissue, which may make it difficult to locate the lesion. For example, typically over 75% of images have no obvious contrast between the lesion and surrounding background tissue. This time period in which there is no obvious contrast frequently occurs during the portal venous phase (PVP), which is problematic as the visibility of the lesion during the PVP is important for lesion diagnosis.

Currently, clinicians often need to repeatedly review the entire loop of CEUS images and make diagnoses according to their own experience and medical knowledge. There are several guidelines (e.g. CEUS-LiRADS, WFUMB-EFSUMB liver CEUS guidelines, China liver CEUS guidelines) assisting clinicians to perform diagnosis based on CEUS loops. However, although there are guidelines for liver lesion diagnosis based on CEUS data, there is no requirement for clinicians follow these guidelines. Different hospitals may use different templates to analyse CEUS loops. Therefore, the accuracy of diagnosis depends heavily on clinicians' personal experience in addition to the particular guidelines and templates that are used, which can cause high inter-operator variance.

It is therefore desirable to improve the consistency of analysis of CEUS images in addition to reducing the time required to analyse CEUS images.

According to an aspect, there is provided an apparatus for use in analysing ultrasound image data of a subject, the apparatus comprising: a memory comprising instruction data representing a set of instructions; and a processor configured to communicate with the memory and to execute the set of instructions, wherein the set of instructions, when executed by the processor, cause the processor to:
obtain contrast-enhanced ultrasound image data comprising a plurality of frames; estimate at least one time point corresponding to at least one event; select a sample of frames from the plurality of frames based on the at least one estimated time point; and classify each of the sample of frames.

This advantageously reduces the time taken to identify important points and features within a CEUS loop which may then be used for diagnosis by a user.

The processor may be further caused to derive a feature associated with at least one event based on the classification of each of the sample of frames. Derivation of features in such a manner may be performed more consistently and independent of (e.g. the skill level of) a user (e.g. a clinician). The estimating of the at least one time point may be based on the CEUS image data.

According to a further aspect, there is provided an ultrasound imaging system comprising the apparatus and an ultrasound transducer with which to obtain the contrast-enhanced ultrasound image data.

According to a further aspect, there is provided a computer implemented method for use in analysing ultrasound image data of a subject, the method comprising: obtaining contrast-enhanced ultrasound image data comprising a plurality of frames; estimating at least one time point corresponding to at least one event; selecting a sample of frames from the plurality of frames based on the at least one estimated time point; and classifying each of the sample of frames.

According to a further aspect, there is provided a computer program product comprising computer readable medium the computer readable medium having computer readable code embodied therein, the computer readable code being configured such that, on execution by a suitable computer or processor, the computer or processor is caused to perform the method described above.

Such methods may allow for improved consistency of analysis of CEUS images in addition to reducing the time required to analyse CEUS images.

In a further example, the estimation of the at least one time point may be based on a rough time intensity curve which is determined by processing a region of each frame at a predefined location relative to the frame to produce a time intensity curve of the plurality of frames and identifying the at least one time point at which an event occurs based on the time intensity curve.

Thus, using a particular portion of each image in the same relative position in each frame, thereby producing a "rough" time intensity curve of the image, the time at which particular events occur can be determined. For example, the time intensity curve may be taken for a region of each frame which is substantially the whole frame, or a portion of the frame over 50% of the area of the frame. For example, the area of the region may be two third of the area of the frame. The region may have a centre which corresponds to the centre of the frame.

It has been determined by the inventors that the use of such a region provides a quick and accurate method for producing a time intensity curve which is then usable to extract time points at which an event occurs. This is particularly advantageous over a system in which a small area is indicated by a user and used to determine occurrence of an event, as it is quicker and does not require user interaction, thereby improving the consistency of the extraction of data.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 an apparatus for use in analysing ultrasound image data of a subject;
Fig. 2 illustrates an example ultrasound system;
Fig. 3 illustrates a method according to an example;
Fig. 4 illustrates a time intensity curve of regions of frames of ultrasound images according to an example;
Fig. 5 illustrates a deep learning model for use in the methods according to an example;
Fig. 6 illustrates the selection of optimal reference frames and the detection of a lesion in the optimal reference frames;
Fig. 7 illustrates a CEUS image with marked events according to an example;
Fig. 8 illustrates flow charts for determining the degree of enhancement of the arterial phase, the portal vein phase, and the late phase;
Fig. 9 illustrates a flow chart for the classification of pattern of enhancement of the arterial phase;
Fig. 10 illustrates a flow chart for the classification of washout type; and
Fig. 11 illustrates a flow chart of a method to process CEUS data.

### DETAILED DESCRIPTION OF EMBODIMENTS

As described above, the apparatus and methods described herein may allow for improved consistency of analysis of CEUS images in addition to reducing the time required to analyse CEUS images.

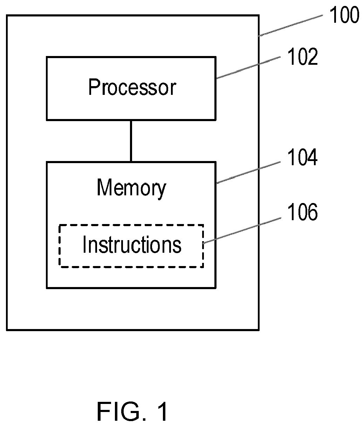

Turning now to Fig. 1, which shows an apparatus 100 for use in analysing ultrasound image data of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

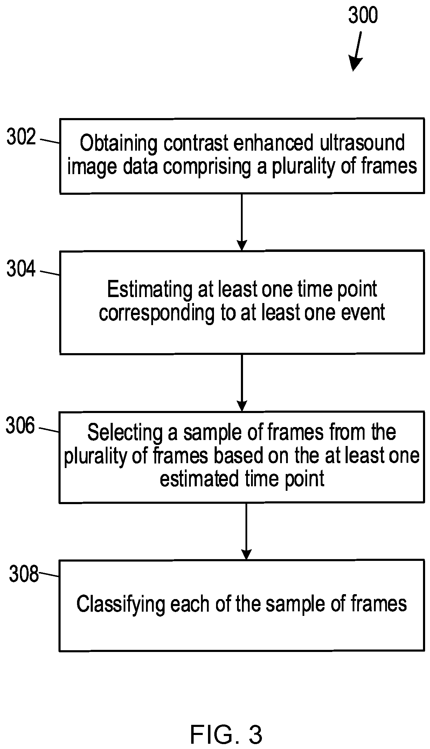

Generally, the apparatus may be adapted or configured to perform any of the embodiments of the methods described herein, such as the method 300 as described below with respect to Fig. 3.

The apparatus comprises a memory 104 comprising instruction data representing a set of instructions 106 and a processor 102 (e.g. processing circuitry or logic) configured to communicate with the memory and to execute the set of instructions 106. Briefly, the set of instructions 106, when executed by the processor 102, cause the processor 102 to obtain contrast-enhanced ultrasound image data comprising a plurality of frames; process a region of each frame at a predefined location relative to the frame to produce a time intensity curve of the plurality of frames; and identify at least one time point at which an event occurs based on the time intensity curve.

As described above, a problem with diagnosing using CEUS images is consistency of extraction of features for analysis. The proposed apparatus herein may enable more consistent extraction of features for diagnosis in addition to reducing time required to extract the features.

In more detail, the processor 102 may comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the sequence of ultrasound image data and/or any other information or data received, calculated or determined by the processor 102 of the apparatus 100 or from any interfaces, memories or devices that are external to the apparatus 100. The processor 102 may be configured to control the memory 104 to store the sequence of ultrasound image data and/or the any other information or data received, calculated, or determined by the processor.

In some embodiments, the memory 104 may comprise a plurality of submemories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 may be comprised in an ultrasound system. For example, an ultrasound system may comprise the apparatus 100 described above, and further comprise a transducer with which to record the sequence of ultrasound image data.

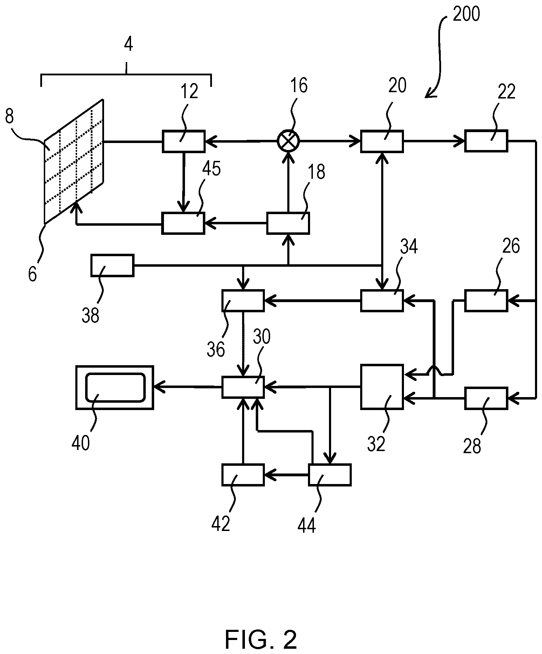

Fig. 2 shows an example ultrasound system 200. The ultrasound system 200 may comprise the apparatus 100 described above. In other embodiments, components of the ultrasound system 200 may be adapted to perform the method 300 described below. The ultrasound system 200 may be adapted to collect the image data for use in the methods described herein.

The system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes.

The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, where instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38, such as the seed points for locating the surface of the muscle, as described in detail below.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The skilled person will appreciate that the detail provided above and the components illustrated in Fig. 2 are an example only and that an ultrasound system may have different components to those illustrated therein.

Turning now back to Fig. 1, and the functionality of the processor 102, as noted above, the processor 102 is caused to obtain contrast-enhanced ultrasound image data comprising a plurality of frames;, estimate at least one time point corresponding to at least one event, select a sample of frames from the plurality of frames based on the at least one estimated time point, and classify each of the sample of frames. The processor may obtain the CEUS image data from an ultrasound system, a processor comprised in an ultrasound system, or a remote server or the like. The processor may be comprised in an ultrasound system.

Turning to Fig. 3, there is a computer implemented method 300 for use in analysing ultrasound image data of a subject. Embodiments of the method 300 may be performed, for example by an apparatus such as the apparatus 100 described above.

Briefly, in a first step 302, the method 300 comprises: obtaining contrast-enhanced ultrasound image data comprising a plurality of frames. In a second step 304, the method 300 further comprises estimating at least one time point corresponding to at least one event. In a third step 306, the method 300 further comprises selecting a sample of frames from the plurality of frames based on the at least one estimated time point. In a fourth step 308, the method further comprises classifying each of the sample of frames.

The estimation of the at least one time point may be based on at least one of: a rough time intensity curve, a conventional time intensity curve, user input. Thus, the time point at which an event occurs can be estimated. The method may further comprise deriving a feature associated with at least one event based on the classification (e.g. the classification result) of each of the sample of frames. The feature associated with at least one event may be derived using a classification algorithm or by receiving input by a user responsive to presenting the classification results to the user.

The feature may be at least one of: a degree of enhancement of a lesion, a pattern of enhancement of a lesion, washout type, early washout, enhancement of a lesion changing from hyper enhancement to iso enhancement, enhancement of a lesion changing from iso enhancement to hypo enhancement. The event may be at least one of: arterial phase onset, arterial phase peak, lesion peak enhancement, arterial phase, portal vein phase, late phase, time from arterial phase onset to lesion peak enhancement.

The sample of frames may be one of: a number of frames around the estimated time point; a number of frames between two estimated time points; frames in a predetermined time period. Thus, an estimated value from a TIC or a pre-determined value (for example, of 2 mins as a typical value for portal vein phase) may be used as the estimated time point. The sample of frames may be a number of frames between e.g. an estimated arterial phase onset and an estimated peak arterial phase. The sample of frames may be a predetermined time period such as the last recorded loop as the time period for late phase.

The method may further comprise that the estimation of the at least one time point is based on a rough time intensity curve which is determined by processing a region of each frame at a predefined location relative to the frame to produce a time intensity curve of the plurality of frames, and identifying the at least one time point at which an event occurs based on the time intensity curve.

Advantageously, the method enables time points to be identified (e.g. using a time intensity curve), which may enable analysis of the plurality of frames of CEUS image data (e.g. a CEUS loop) to identify and extract features. The extraction of features may be performed by a user using the extracted time points or may be performed by a processor. Extraction of features may be made more consistently and independent of (e.g. the skill level of) a user (e.g. a clinician) using the methods described herein. Such a method using image processing is further advantageous as it reduces the time taken to identify important points within a CEUS loop which may then be used for diagnosis by a user.

In an example, the method is performed to analyse a lesion in a liver of a subject. However, it will be appreciated that the methods described herein may be equally applicable in other applications, such as for analysing ultrasound image data of a thyroid or breast of a subject.

In the examples herein, CEUS image data of a subject has been gathered by a clinician. The CEUS image data may comprise the loops recorded by the clinician. In an example, the clinician may identify a focal liver lesion of interest of a subject, and acquire the 2D CEUS data of the lesion. The clinician may hold the probe on the same plane and record a 2D loop for around 2 minutes after the injection of contrast agent. After that, the clinician may record several smaller loops from 2 minutes to 6 minutes. The recorded CEUS image data may then be obtained by the processor.

In order to estimate the at least one time point corresponding to at least one event, in one example, a "rough" TIC curve may be used. In particular, a rough time intensity curve may be used which is determined by processing a region of each frame at a predefined location relative to the frame to produce a time intensity curve of the plurality of frames and identifying the at least one time point at which an event occurs based on the time intensity curve.

The region of the frame may be at a predetermined location relative to the frame and may be a set portion of the frame. For example, the same predetermined region may be used for each frame without requiring input of a user to indicate the location of a region to be used. Thus, the position of a region may be pre-set. The area of the region may be a set proportion of the frame. For example, the region may be at least half the area of the frame. This area may be used as there is a high likelihood that the object of interest, such as a liver lesion, will be present in an area that covers half of the frame, as typically a user will attempt to centre the lesion when performing the CEUS imaging. The frame may be a least two thirds of the area of a frame which may provide an improved result. The area may be substantially the whole frame. A centre of the region may correspond to a centre of the frame. Analysis of these regions may provide a "rough" TIC, where intensity of a large portion of an image (including a lesion) may be used to roughly determine the intensity of a lesion over time. It has been determined by the inventors that a "rough" TIC provides a sufficiently good indication of the intensity of the lesion over time, and can be used to determine time points at which events occur.

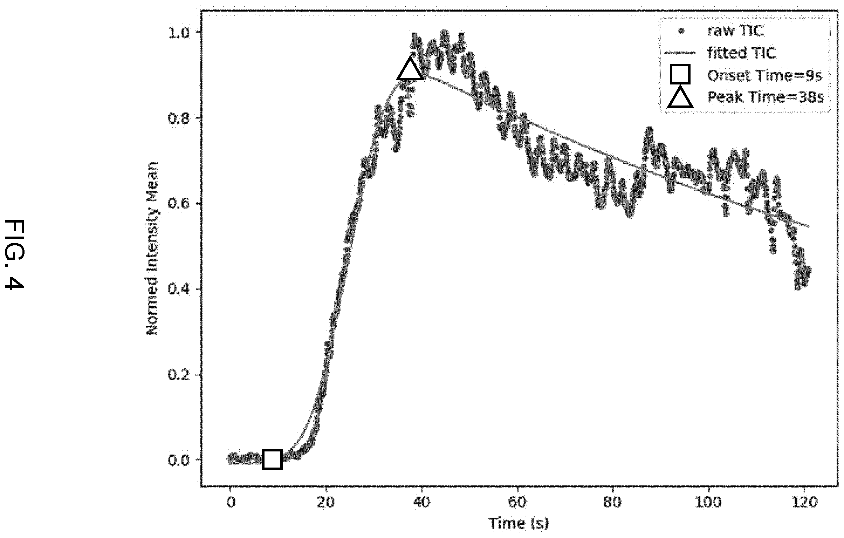

Fig. 4 illustrates a time intensity curve (TIC) of CEUS image data relating to a plurality of frames taken of a liver of a subject after an injection of contrast. The raw TIC curve comprising the plurality of points in the image illustrates a normed intensity mean of each frame (raw data points indicated as circular points in the image). The TIC curve is fit to reduce the impact of noise and outliers and is illustrated as the line passing through points in the image. The fitting may be performed using a low-pass filter or lognormal model, for example. In the example of Fig.4, the raw TIC curve is calculated using a region two thirds of the size of a frame, and wherein the centre of the region coincides with the centre of a frame.

The TIC curve may be used to determine time points at which events occur. In this particular example, as the frames taken are frames of a liver lesion, the TIC curve of these frames may be used to determine the liver lesion's enhancement at different time points after the injection of contrast. In particular, the time at which events such as onset of arterial phase (AP) and peak arterial phase can be determined using the TIC curve.

The arterial phase (AP) onset time (illustrate by a square point in the graph) can be determined as the time at which the intensity begins to increase (an initial increase in gradient) in the CEUS image data, and the AP peak time (indicated by a triangular point in the graph) can be determined as the time of peak intensity, or by determining the time at which the intensity begins to decrease (an initial decrease in gradient) after the initial increase. Thus, the TIC curve may be used to determine an event of arterial phase onset and an event of arterial phase peak at particular time points (corresponding to particular frames). In this particular example, the AP onset time is determined to be at 9 seconds and the AP peak time is determined to be at 38 seconds.

The identified time points of the time intensity curve may be used to determine an event of lesion peak enhancement, where the lesion is most distinct from the surrounding tissue. The lesion peak enhancement may occur at some time between AP onset time and AP peak time. Using the aforementioned time points extracted from the TIC curve, it may be possible to also select the best frame(s) for lesion diagnosis, or derive enhancement degree/patterns for particular phases which occur between time points or events.

It will be appreciated that the "rough" TIC curve described above is a particularly advantageous method with which to estimate the at least one time point corresponding to at least one event, however, it will be appreciated that other methods, such as using a conventional time intensity curve, may equally be used. The time point may be indicated by a user.

An optimal reference frame may demonstrate maximum contrast between the lesion and its surrounding background tissue. An optimal frame with good contrast between a lesion and its surrounding background tissue normally occur during the initial arterial phase (AP), especially from the AP onset time to the AP peak time. The arterial phase onset time and the arterial phase peak time may be determined using the TIC as described above. The time at which the optimal reference frame occurs may be determined by multiplying a ratio to the difference between the onset arterial phase time and the peak arterial phase time, and adding the result to the onset arterial phase time (e.g. time of optimal reference frames is equal to AP onset time plus ratio * (AP peak time - AP onset time)). The ratio is, for example, (0, 1). An optimal ratio may be predetermined and may be a set value which typically provides acceptable results (e.g. during experimentation where different ratios are used and the ratio which produces optimal reference frames is determined as being the optimal ratio). For example, a ratio set as 40% may provide an acceptable result. The optimal ratio may be determined using a machine learning model trained with a training dataset (e.g. a training dataset of sets of frames between AP onset time and AP peak time which have been labelled to indicate at what ratio the optimal reference frames occur) to assess at what ratio a frame with best contrast between a lesion and surrounding material occurs (e.g. by inputting the frames between AP onset time and AP peak time to the machine learning model).

The optimal reference frame may be processed to locate a lesion. In particular, the method may further comprise steps of: determining an optimal reference frame, the optimal reference frame being at a time a predetermined ratio of the difference between a time of arterial phase onset and a time of arterial phase peak after the arterial phase onset; and processing the optimal reference frame to locate the lesion.

Optimal reference frames may comprise the optimal frame, and may comprise frames adjacent to the optimal frame. These frames may also have acceptable contrast. The time period at which the optimal reference frames occur may be determined by multiplying a first ratio to the difference between the onset arterial phase time and the peak arterial phase time, and adding the result to the onset arterial phase time, and by multiplying a second ratio to the difference between the onset arterial phase time and the peak arterial phase time, and adding the result to the onset arterial phase time. The frames between these two points may be optimal reference frames which also have acceptable contrast. For example, these frames may be between AP onset time + ratio 1*(AP peak time - AP onset time) and AP onset time + ratio2^{*}(AP peak time - AP onset time). The first ratio (ratio1) and the second ratio (ratio2) may also be obtained by experimentation as described above in relation to the ratio for the optimal frame. As an example, ratio1 set as 30% and ratio2 set as 50% have been found by the inventors to provide an acceptable result based on experimentation.

The optimal reference frames may be processed to locate a lesion. In particular, the method may further comprise steps of: determining optimal reference frames, the optimal reference frames being at a time period between predetermined ratios of the difference between a time of arterial phase onset and a time of arterial phase peak after the arterial phase onset; and processing the optimal reference frames to locate the lesion.

The locating of the lesion may be implemented by segmenting the images using any appropriate conventional image processing method such as the one described in T.F.Chan, L.A.Vese, "Active contour without edges", IEEE transactions on image processing 10, February 2001, using neural networks or a deep learning method such as the one described in Ronneberger, Olaf; Fischer, Philipp; Brox, Thomas. "U-Net: Convolutional Networks for Biomedical Image Segmentation". arXiv: 1505.04597, 2015. In the following example, deep learning was used.

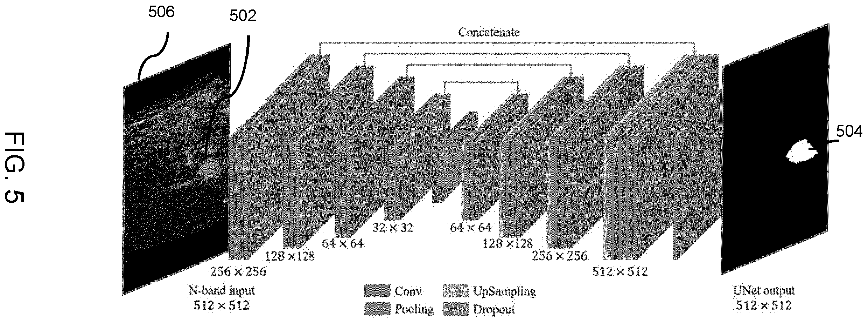

Fig. 5 illustrates a deep learning model (see, for example, Ronneberger, Olaf; Fischer, Philipp; Brox, Thomas. "U-Net: Convolutional Networks for Biomedical Image Segmentation". arXiv: 1505.04597, 2015) where lesion segmentation is applied to distinguish the lesion 502, 504 from the surrounding tissue. Segmentation may be applied to the optimal reference frame, or to at least one frame of the optimal reference frames 506. It will be appreciated that any algorithm capable of segmenting the lesion may be used in the method. By applying segmentation to optimal reference frames, the time taken to identify and segment a lesion may be reduced, as the frames for use in segmentation have been selected so that the segmentation is performed on images having the best contrast between the lesion and the surrounding tissue.

After segmentation, a point inside the lesion and/or a boundary or bounding box of a lesion found in the optimal reference frame (or the optimal reference frames) can also be indicated on other frames of the image data. For example, the location of the lesion in the optimal frames can be shown in each frame of the image data. Therefore, when a user is reviewing the image data they can understand where the lesion is located even when the distinction between the lesion and the surrounding material is not particularly visible to the user. This may assist a user in making a more accurate assessment of the lesion for diagnosis.

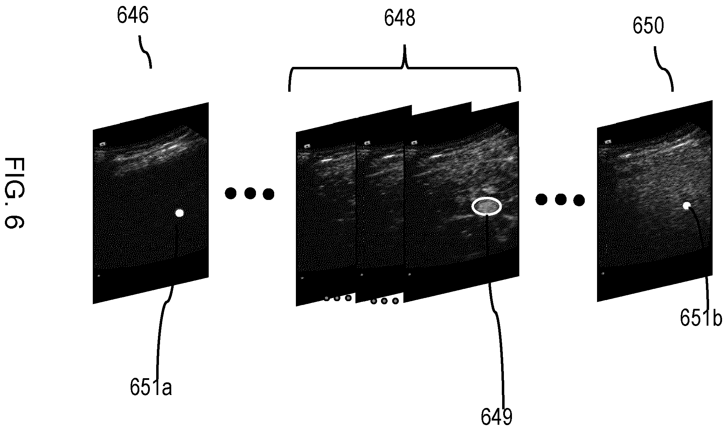

An example of this is illustrated in Fig. 6 which shows the selection of optimal reference frames 648 and a detected boundary 649 of a lesion in the optimal reference frames 648, where a visual marker of the lesion 651 a, 651b is shown in the first frame 646 and the last frame 650 (it will be appreciated that the visual marker 651 may be present on other frames between the first frame 646 and the last frame 650 which are not shown in this image, and may instead be present on frames which are not the first frame and the last frame). A visual marker 651a, 651b of the lesion may be in the form of a point inside the lesion region or the boundary of lesion region or a bounding box covering lesion region. As can be seen in this image, it is not possible in this example to clearly see the position of the lesion in the first and last frame, and therefore an indication of the location of the lesion may be helpful for a clinician or user to understand where the lesion is in the image, so that they are able to interpret the images to make a diagnosis.

Alternatively, or additionally, the location of the lesion in the optimal reference frames can be used to determine the location of the lesion in frames which are not the optimal reference frames. For example, there may be small, minor visual changes of lesion between adjacent frames. Refinement of the location of the lesion may therefore be desirable in order that the indicated location of the lesion matches with the actual location of the lesion in a particular frame. The location of the lesion in the optimal reference frames may be used to indicate a predicted location of the lesion in frames which are not the optimal reference frames (for example, indicating a boundary of a lesion or the like). The predicted location may then be used to determine the actual location of the lesion in the frames which are not the optimal reference frames (for example, the actual boundary of a lesion in a particular frame which is not an optimal reference frame).

In particular, the position of the lesion on a current frame (for example, a frame being observed by a user) can be displayed by determining the position of the lesion on multiple frames of the image data using deep learning or conventional image processing methods. In an example of conventional image processing, the boundary of a lesion of the previous frame adjacent to the current frame is set as the initial boundary in the current frame, and then the boundary of the lesion is fine-tuned on the current frame by a level set based method such as the one described in T.F.Chan, L.A.Vese. "Active contour without edges". IEEE transactions on image processing 10, February 2001. Thus, the boundary of the lesion as found for a previous frame may be edited to fit the actual boundary of the lesion in a current frame. The boundary of the lesion may be displayed in each frame.

Each of the sample of frames are classified. In particular, each of the sample of frames may have their degree of enhancement or pattern of enhancement classified.

The method may further comprise a step of classifying a degree of enhancement of a lesion for each of a sample of frames of the plurality of frames and/or a pattern of enhancement of a lesion for each of a sample of frames of the plurality of frames. The sample of frames may be all the frames or may be a number of frames less than the plurality of frames. For example, the sample of frames may comprise frames spaced 1 second apart. The sample of frames may be a (predetermined) number of frames around the estimated time point; a (predetermined) number of frames between two estimated time points; frames in a predetermined time period.

The location of the lesion may be determined by the methods described above, or may be indicated by a user.

Each of the sample of frames may be analysed using conventional image processing methods or deep learning methods (such as pre-trained neural networks) in order to classify each of the sample of frames. For example, the classification of each of the sample of frames may comprise determining the degree of enhancement/pattern of enhancement of the lesion in the image (e.g. using the indicated location of the lesion in the image and determining the degree of enhancement or pattern of enhancement of the lesion relative to the surrounding tissue). Each frame may be compared to a predicted result of a degree of enhancement of a frame, and based on the comparison, a value or classification of the degree of enhancement may be assigned to the frame.

A machine learning algorithm which has been trained using a training data set may be used in order to classify the degree of enhancement of the lesion. The training data set may be labelled loops of frames which show hyper, iso and hypo enhancement. The trained algorithm may take as input a loop between AP onset time and Peak time to predict AP degree of enhancement, a loop between 115s and 120s to predict PVP degree of enhancement, and the last frames of the plurality of frame to predict LP degree of enhancement A value may be assigned to each frame indicating the degree of enhancement of the lesion relative to the tissue, from a scale of 0 to 1, 0 being no enhancement of the lesion relative to the tissue and 1 being high enhancement relative to the tissue.

Alternatively, each frame may be classified into one of hyper enhancement (where enhancement of the lesion is greater than the enhancement of the surrounding tissue), iso enhancement (where enhancement of the lesion is very similar to the enhancement of the surrounding tissue), or hypo enhancement (where enhancement of the lesion is less than the enhancement of the surrounding tissue), for example using a machine learning algorithm which has been trained using a training data set in order to classify the enhancement of the lesion. Each frame may be analysed to determine the likelihood that a frame corresponds to a class of at least one of hyper enhancement, iso enhancement and hypo enhancement. A value between 0 and 1 for each class may then be assigned to each frame indicating the likelihood of the frame corresponding to the class. The class with the highest likelihood may be determined as the classification of enhancement for a frame.

The results of frames may then be used to derive a feature associated with at least one event based on the classification result of each of the sample of frames. For example, results of the frames may be combined to determine the degree of enhancement for a particular set of frames, for example in a particular phase (e.g. corresponding to a particular event). The combining may be performed by any means such as a mean calculation, majority vote etc.. For example, where a final result is [0.1, 0.3, 0.6] for [hyper, iso, hypo], then the lesion may be predicted to have hypo enhancement with a confidence of 0.6. The degree of enhancement for the sample of frames may be used to determine at least one time point at which an event occurs. For example, the event may be the enhancement of a lesion changing from hyper enhancement (where enhancement of the lesion is greater than the enhancement of the surrounding tissue) to iso enhancement (where enhancement of the lesion is very similar to the enhancement of the surrounding tissue), or enhancement of a lesion changing from iso enhancement to hypo enhancement (where enhancement of the lesion is less than the enhancement of the surrounding tissue). Changes in the enhancement of a lesion may therefore be determined from the determined degree of enhancement of a sample of frames, and these changes (e.g. the features) correlated to events and time points in the data. For example, the determined enhancement may be an indication of the enhancement of the lesion relative to the surrounding tissue. Therefore, from a set of determined enhancement for a set of frames (e.g. classifications), it may be possible to tell at what time point the enhancement of the frames changes from one sort of enhancement to another (e.g. hyper enhancement, iso enhancement, or hypo enhancement). The change of classification of enhancement from hyper enhancement, iso enhancement, or hypo enhancement to another of the enhancement categories in the sequence of frames which correlates to time points may indicate a time point at which an event occurs.

Thus, a plurality of time points (corresponding to events) may be estimated using the methods above. For example, any of arterial phase onset time, arterial phase peak time, the time when the lesion has peak enhancement, the time when the lesion changes from hyperenhancement to iso-enhancement and the time when the lesion changes from iso-enhancement to hypo-enhancement may be determined. The event may be one of arterial phase onset, arterial phase peak, lesion peak enhancement, arterial phase, portal vein phase, late phase, time from arterial phase onset to lesion peak enhancement. Any of these time points may be indicated in a user interface at the relevant point in the CEUS data (for example, in relation to a frame corresponding to the relevant time point).

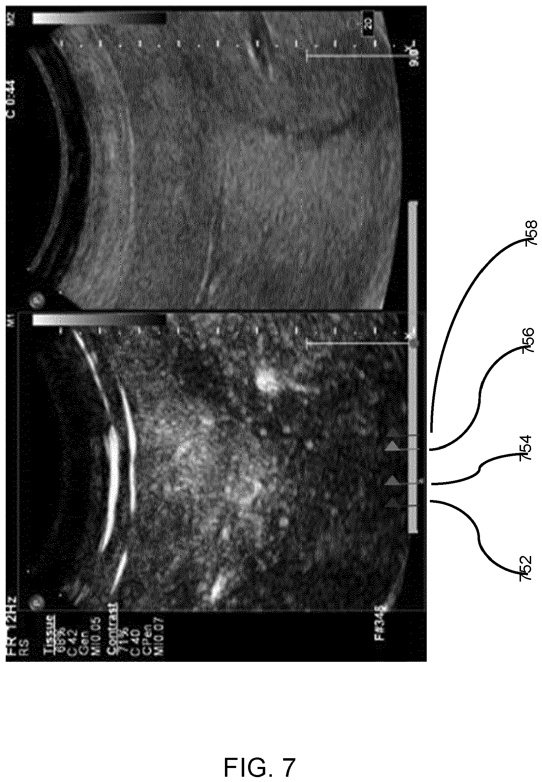

Fig. 7 illustrates an example of the CEUS data where time points of AP onset time 752, peak enhancement 754, change to iso-enhancement 756 and change to hypo-enhancement 758 have been indicated. It will be appreciated that visual markers (such as those shown in Fig. 7) may indicate the time point in a loop where an event occurs on a display, so that a user (such as a clinician) can easily find the relevant points in order to view the liver lesion at these points. This may therefore reduce the time taken for a user to analyse CEUS data.

The method may further comprise extracting further information from the CEUS data using the determined time points. A feature associated with at least one event based on the classification result of each of the sample of frames may be derived. For example, the classification of each frame may be used to derive a feature associated with at least one event, where the sample of frames have been selected based on the event. The feature associated with at least one event may be derived using a classification algorithm or by receiving input by a user responsive to presenting the classification results to the user. The feature may be any of a degree of enhancement of a lesion, a pattern of enhancement of a lesion, washout type, early washout, enhancement of a lesion changing from hyper enhancement to iso enhancement, enhancement of a lesion changing from iso enhancement to hypo enhancement.

A classification algorithm may be used to derive the degree of enhancement or the pattern of enhancement for a particular set of frames corresponding to at least one time point, such as the frames belonging to a phase. Any suitable classification algorithm may be used. The classification algorithm may derive the degree of enhancement of a lesion or the pattern of enhancement of a lesion of a set of frames corresponding to at least one time point based on the classified degree of enhancement of a lesion or the pattern of enhancement of a lesion of the sample of frames. For example, the degree of enhancement/pattern of enhancement may be classified for a large portion of the CEUS image data, where then a subset of frames are selected corresponding to a relevant time point, and the degree of enhancement/pattern of enhancement of the subset of frames may then be used to derive the degree of enhancement/pattern of enhancement at that time point. The classification algorithm may use the determined or classified degree of enhancement for each of a plurality of frames belonging to a phase to derive the degree of enhancement for a particular phase (for example, to derive a feature associated with at least one event). The classification algorithm may use the determined or classified pattern of enhancement for a plurality of frames belonging to the phase to classify the pattern of enhancement for a particular phase, thereby determining the pattern of enhancement for a particular phase.

Thus, classification algorithms may be used to process frames associated with at least one event (e.g. belonging to particular phases) in order to derive the degree of enhancement or pattern of enhancement for various phases. A classification algorithm may be any of logistic regression, k-nearest neighbours, decision trees, support vector machines or native bayes, for example. In an example, decision trees may be used as the classification algorithm (however it will be appreciated that any other appropriate classification algorithm may be used). To derive a feature associated with a phase, the classification of frames relevant to the phase may be used. The frames around a particular time point may be used to derive a feature associated with a phase by determining the number of frames with a particular classification result (the classification result being a degree of enhancement), where the classification having the highest number of frames is determined as the degree or pattern of enhancement of a phase.

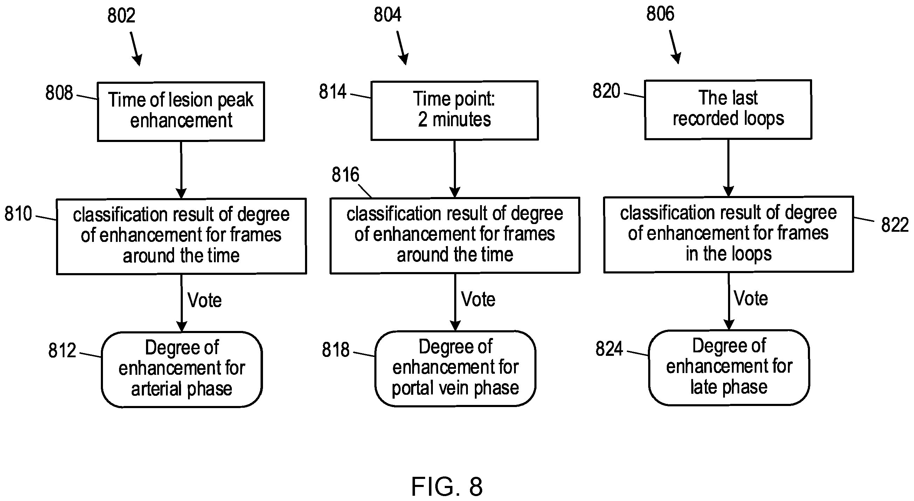

Fig. 8 illustrates a method of deriving a feature associated with an event (in this example the derivation of the degree of enhancement for arterial phase 802, portal vein phase 804, and late phase 806). For example, using a time of lesion peak enhancement 808 determined using the methods described above, the classification result of the degree of enhancement for frames around the time of lesion peak enhancement (e.g. a predetermined number of frames around the time) can be determined 810, and the enhancement for arterial phase may then be determined 812. In particular, the class with the most frames is determined as being the class corresponding to a degree of enhancement of the phase. The classes may be, for example, hyper enhancement, iso enhancement, or hypo enhancement. Similarly, the degree of enhancement for portal vein phase 818 may be determined by selecting a time point of 2 minutes 814 and classifying the result of degree of enhancement for a predetermined number of frames around the time point of 2 minutes 816, and determining the number of frames in each class. The class with the most frames is the derived feature (corresponding to a degree of enhancement) of the portal vein phase. The degree of enhancement for late phase 806 may be determined using a predetermined number of the last frames in the CEUS data 820 (e.g. the last recorded loop(s)), and classifying a degree of enhancement for these frames 822. The class with the most frames is determined as being the derived feature (degree of enhancement) of the late phase 822. Thus, the feature of degree of enhancement is derived for an event of late phase.

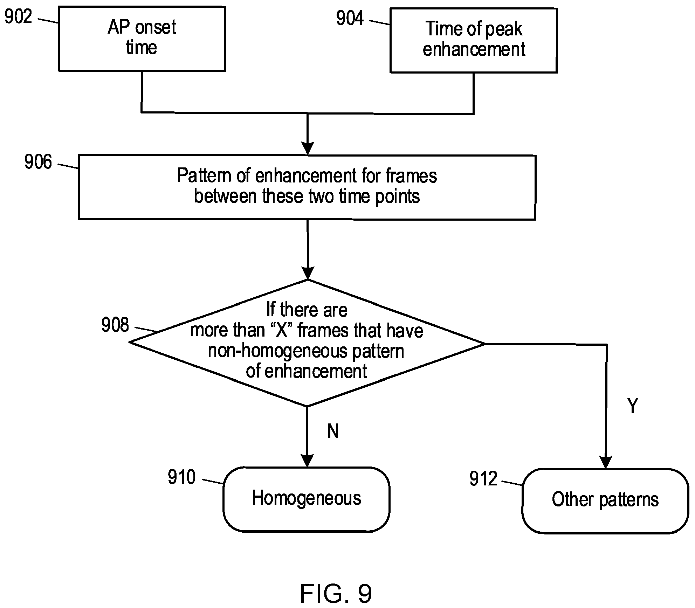

The method may also comprise determining the pattern of enhancement for a phase. In particular, the method may comprise determining the pattern of enhancement for the arterial phase. An example of a flow chart of such a method is illustrated in Fig. 9. For example, the method may comprise selecting frames between a time of arterial phase onset 902 and a time of lesion peak enhancement 904 (using the time points determined using the methods described above) and classifying a pattern of enhancement of each of the selected frames 906 (or a sample of the frames). For example, each of the selected frames may be classified as a pattern of enhancement, such as , homogeneous, non-homogeneous (e.g. heterogenous, peripheral globular/nodular and rim), The classifications may be classifications used for liver lesion diagnosis (for example, according to Li-RADs guidelines). The classification may be performed using any appropriate image processing methods or machine learning algorithms. Each frame may be analysed to determine the likelihood that a frame corresponds to a class of at least one of homogeneous, heterogenous, peripheral globular/nodular and rim. A value between 0 and 1 for each class may then be assigned to each frame indicating the likelihood of the frame corresponding to the class. The feature associated with the phase may then be derived. For example, the class with the highest likelihood may be determined as the pattern of enhancement for a frame. Eeach frame between AP onset time and AP peak time may be assessed to determine the confidence of different classifications such as homogeneous, heterogenous, peripheral globular/nodular and rim, where a number between 0 and 1 indicates the confidence of each class. For example, where the classification indicates values of [0.1, 0.2, 0.3, 0.6], 0.1 indicates homogeneous enhancement confidence, 0.2 indicates heterogenous enhancement confidence, 0.3 indicates peripheral globular/nodular confidence and 0.6 indicates rim confidence. As rim has the highest confidence, it may be determined that this is the classification for the frame. The results of frames within a time period may then be combined as described above, using a mean calculation, majority vote etc to determine the pattern of enhancement of a phase (e.g. the feature associated with the event), such as between AP onset time and AP peak time.

The method may further comprise determining if there are more than a predetermined number of frames that are non-homogeneous 908. If there are more than a predetermine number of frames (YES), the method may determine the pattern of enhancement for arterial phase as non-homogeneous 912. If there are not more than a predetermine number of frames (NO), the method may determine the pattern of enhancement for arterial phase as homogeneous 910. Thus, the method may determine the pattern of enhancement for arterial phase (e.g. as either homogeneous 910 or as another pattern (e.g. heterogenous (non homogeneous), peripheral globular/nodular and rim) 912) - thereby deriving a feature associated with an event.

The method may further comprise assessing washout, which is a reduction in enhancement of a lesion relative to the surrounding tissue from an earlier to a later phase, resulting in hypo-enhancement at any time. The method may further comprise determining whether early washout presents by determining if the enhancement of the lesion changes from iso enhancement to hypo enhancement at a time point before a first predetermined time period (for example, 60 seconds) based on the degree of enhancement of the lesion for each of a sample of frames. For example, in one method, if the degree of enhancement of the arterial phase is hyper or iso enhancement (as determined by the methods above), and the degree of enhancement at 60s is hypo enhancement, then it may be determined that early washout presents. This method may use a pre-trained model as described above in relation to the determination of the degree of enhancement to determine the degree of enhancement of frames. Early washout may also be predicted (either a YES or a NO) using frames between AP onset time and 60s and a trained model, which may be trained to classify the washout type. The method may also derive washout type at a second predetermined time period (for example, 120 seconds) if early washout presents using the determined degree of enhancement or using a pre-trained neural network (or any other appropriate image processing or machine learning techniques) to classify the washout type of each of a predetermined number of frames around the second predetermined time point and by determining the number of frames in each washout type class (the classes being marked, mild, and none), thereby deriving the washout type.

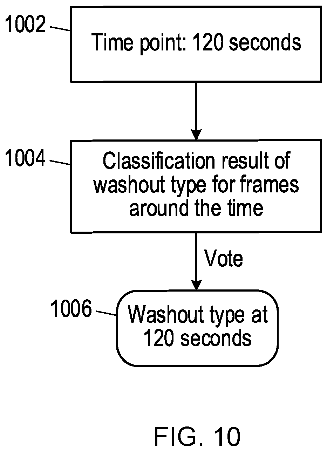

Fig. 10 illustrates a flow chart of a process for determining the washout type at 120 seconds. For a time point of 120 seconds 1002, the classification result of washout type (marked, mild, and none) for frames around the time point 1004 (e.g. a predetermined number of frames around the time point) are obtained. For example, a model may be trained using sets of frames which have been classified, then the trained model may be used to predict the classification of an input set of frames. In particular, a feature associated with at least one event based on the classification result of each of the sample of frames may be derived. The washout type at 120 seconds is then determined 1006 by assessing how many frames are in each class (e.g. by voting), where the class with the highest number of frames is corresponds to the washout type.

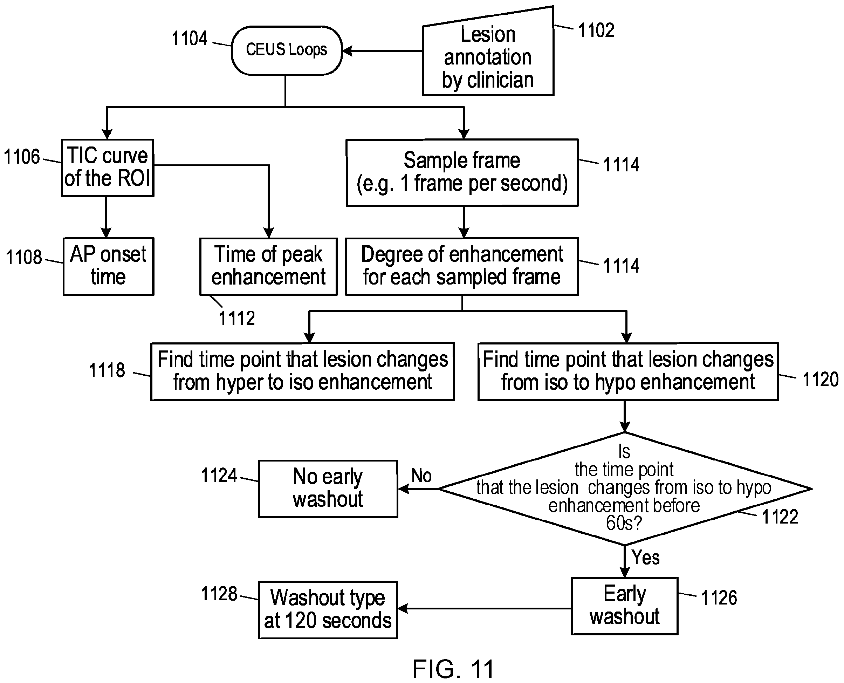

An overview of a method which may be used to process CEUS data, and may comprise some or all of the steps described above, is illustrated in Fig. 11, where a flow chart of steps of the method is shown. In particular, this Figure illustrates a first step of a lesion annotated by a user 1102 (however, it will be appreciated that the lesion may instead be detected using the methods described above), after which CEUS data (in the form of loops) are obtained 1104. Using the methods described above, a TIC curve based on a predetermined region of each frame may be obtained 1106 from the CEUS data. Using the TIC curve, the AP onset time 1108 and the time of peak enhancement of the lesion 1112 may be estimated. A sample of frames may be taken from the CEUS data 1114 (for example, 1 frame per second). A degree of enhancement of the lesion may be determined for each frame of the sample 1116 using the methods described above. Using the degree of enhancement of the sampled frames, the time point at which the lesion changes from hyper to iso enhancement 1118 may be estimated, and the time point at which the lesion changes from iso to hypo enhancement 1120 may also be estimated. It may then be determined whether the time point at which the lesion changes from iso to hypo enhancement occurs before 60 seconds 1122. If it does not (NO), it may be derived that no early washout occurs 1124. If it does (YES), it may be derived that early washout occurs 1126. If early washout occurs, the washout type at a time point of 120 seconds may then be derived 1128 using the methods described above.

It is noted that the flow chart of Fig. 11 is illustrative of a combination of functions that may be performed to analyse or process the CEUS data using the methods described herein, however, the method may include any of the steps of the method outlined above in any appropriate combination. Furthermore, a user may select the results, events and/or time points that they want the method to determine, and the method may determine those results.

Any of the events, results and/or time points determined by any of the methods described above may be presented to the user and/or annotated on an CEUS data which is being considered by the user. The detection results may be displayed, may be stored, and/or a user may be asked for confirmation of any of the events or derived features. The results of the methods such as the time of events, classification results or determinations, may be collated and used to form the basis of a report. A user may select which of the results are included in a report. The user may adjust any of the results, events, derived features and/or classifications before the result is compiled.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solidstate medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for use in analysing ultrasound image data of a subject, the apparatus (100) comprising:
a memory (104) comprising instruction data representing a set of instructions (106); and
a processor (102) configured to communicate with the memory (104) and to execute the set of instructions (106), wherein the set of instructions (106), when executed by the processor (102), cause the processor (102) to:
obtain contrast-enhanced ultrasound image data comprising a plurality of frames (100);
estimate at least one time point corresponding to at least one event; select a sample of frames from the plurality of frames based on the at least one estimated time point; and
classify each of the sample of frames.

2. The apparatus (100) as claimed in claim 1, wherein the processor is further caused to derive a feature associated with at least one event based on the classification of each of the sample of frames.

3. The apparatus (100) as claimed in claim 2, wherein the feature associated with at least one event is derived using a classification algorithm or by receiving input by a user responsive to presenting the classification of the frames to the user.

4. The apparatus (100) as claimed in claim 2 or 3, wherein the feature is at least one of: a degree of enhancement of a lesion, a pattern of enhancement of a lesion, washout type, early washout, enhancement of a lesion changing from hyper enhancement to iso enhancement, enhancement of a lesion changing from iso enhancement to hypo enhancement.

5. The apparatus (100) as claimed in any preceding claim, wherein the estimation of the at least one time point is based on at least one of: a rough time intensity curve, a time intensity curve, user input.

6. The apparatus as claimed in any preceding claim, wherein the estimation of the at least one time point is based on a rough time intensity curve which is determined by processing a region of each frame at a predefined location relative to the frame to produce a time intensity curve of the plurality of frames; and
identifying the at least one time point at which an event occurs based on the time intensity curve (306).

7. The apparatus as claimed in any preceding claim. wherein the sample of frames is one of: a number of frames around the estimated time point; a number of frames between two estimated time points; frames in a predetermined time period.

8. The apparatus (100) as claimed in any preceding claim, wherein the event is at least one of: arterial phase onset, arterial phase peak, lesion peak enhancement, arterial phase, portal vein phase, late phase, time from arterial phase onset to lesion peak enhancement.

9. The apparatus (100) as claimed in any preceding claim, wherein the processor (102) is further caused to classify the degree of enhancement of a lesion of the sample of frames, and determine whether early washout presents by determining if the enhancement of the lesion changes from iso enhancement to hypo enhancement at a time point before a first predetermined time period based on the degree of enhancement of the lesion of the sample of frames; and optionally classify washout type at a second predetermined time period if early washout presents using a classification algorithm to derive washout type of frames around the second predetermined time period based on a determined washout type of the frames around the second predetermined time period.

10. The apparatus (100) as claimed in any preceding claim, wherein the processor (102) is further caused to indicate to a user interface the at least one time point in relation to the contrast-enhanced ultrasound image data.

11. The apparatus (100) as claimed in any preceding claim, wherein the processor (102) is further caused to detect a location of a lesion by:
determining an optimal reference frame, the optimal reference frame being at a time a predetermined ratio of the difference between a time of arterial phase onset and a time of arterial phase peak after the arterial phase onset; and
processing the optimal reference frame to locate the lesion.

12. The apparatus (100) as claimed in claim 11, wherein the processor (102) is further caused to use the location of the lesion in the optimal reference frames to indicate a predicted location of the lesion in frames which are not the optimal reference frames, and wherein the processor (102) is further caused to use the predicted location to determine the actual location of the lesion in the frames which are not the optimal reference frames.

13. An ultrasound imaging system comprising the apparatus (100) of any one of the preceding claims and an ultrasound transducer with which to obtain the contrast-enhanced ultrasound image data.

14. A computer implemented method for use in analysing ultrasound image data of a subject, the method comprising:
obtaining contrast-enhanced ultrasound image data comprising a plurality of frames;
estimating at least one time point corresponding to at least one event;
selecting a sample of frames from the plurality of frames based on the at least one estimated time point; and
classifying each of the sample of frames.

15. A computer program product comprising computer readable medium the computer readable medium having computer readable code embodied therein, the computer readable code being configured such that, on execution by a suitable computer or processor (102), the computer or processor (102) is caused to perform the method as claimed in claim 14.
